# EUROPEAN PATENT SPECIFICATION

(11) **EP 1 394 686 B1**
(45) Date of publication and mention of the grant of the patent: **25.07.2007**
(21) Application number: 02019452.8
(22) Date of filing: 30.08.2002
(51) Int. Cl.: G06F 17/00, A61B 5/00, A61B 1/00

(54) **Endoscopic image filing system**
Akquisitionssystem für endoskopische Bilder
Système pour l' acquisition des images endoscopiques

(43) Date of publication of application: 03.03.2004
(73) Proprietor: Olympus Corporation, Shibuya-ku, Tokyo (JP)
(72) Inventor: Shibata, Hiroyuki, Yokohama-shi, Kanagawa (JP)
(74) Representative: von Hellfeld, Axel

(56) References cited:
- US-A- 5 029 016
- US-A- 5 111 306
- US-A- 5 877 819
- US-A1- 2002 115 916
- PATENT ABSTRACTS OF JAPAN vol. 1999, no. 01, 29 January 1999 (1999-01-29) & JP 10 272093 A (GE YOKOGAWA MEDICAL SYST LTD), 13 October 1998 (1998-10-13)
- PATENT ABSTRACTS OF JAPAN vol. 2000, no. 21, 3 August 2001 (2001-08-03) & JP 2001 104246 A (OLYMPUS OPTICAL CO LTD), 17 April 2001 (2001-04-17)

## Description

### BACKGROUND OF THE INVENTION

### 1. Field of the Invention

The present invention relates to an endoscopic image filing system for recording endoscopic images, patient information, and the like, and for recording patient biological information measured by means of a patient monitoring apparatus or similar in the course of an endoscopic examination.

### 2. Description of the Related Art

An endoscope apparatus as used conventionally is constituted such that imaging means are provided in an endoscope which performs observation when a long and narrow insert portion thereof is inserted into an examination site such as a body cavity and constituted such that images of the examination site picked up by the imaging means, that is, endoscopic images, are displayed on a monitor.

In recent years, endoscopic image filing systems, for which an image filing apparatus for recording endoscopic images is connected with the endoscope apparatus, have been widely used.

An endoscopic image filing system is constituted such that upon pressing an endoscope switch, for example a release switch, with which the endoscope apparatus is provided, still images of endoscopic images displayed on the monitor are recorded by the image filing apparatus.

Further, the endoscopic image filing system is not only capable of recording endoscopic images but is also capable of recording a variety of information relating to an endoscopic examination including a physician's view with regard to recorded endoscopic images, patient information such as the age and gender of the patient, as well as the date and time when the endoscopic examination was conducted.

Meanwhile, when the patient's condition during an endoscopic examination is also monitored and an abnormality occurs in the patient, appropriate equipment must be chosen immediately. There are, for example, methods for measuring the patient's maximum blood pressure, minimum blood pressure and pulse using a non-invasive blood pressure manometer. Here it is possible to set the time interval for the blood pressure manometer and to repeat the measurement at fixed intervals. Methods also exist for measuring the arterial blood oxygen concentration and pulse using a pulse oximeter. There are also methods for measuring an electrocardiogram using an electrocardiogram monitor, and also methods for monitoring through integration of the above-mentioned biological information by means of a patient monitoring apparatus that integrates the functions of a blood pressure manometer, a pulse oximeter, and an electrocardiogram monitor.

Methods for displaying a plurality of biological information simultaneously and for recording biological information as digital data were disclosed in European Patent No. EP 0 676 709 A2 and Japanese Patent Application (Kokai) No. H3-258240 and so forth. Such methods could also be employed in an endoscopic examination or during a surgical operation and made it possible to record the condition of the patient undergoing surgery.

Such biological information is not only used to provide information on the condition of the patient in an examination but is also important data for reference at the time of making a diagnosis from examination results. However, although conventional endoscopic image filing systems have permitted the recording of endoscopic images in the course of an endoscopic examination, such systems have not enabled biological information on a patient in an examination to be recorded so as to be related with these endoscopic images.

Other prior art documents include US 5 877 819, US 5 111 306, US 5 029 016, JP 10 272093, JP 2001-104246 and US 2002/115916.

### SUMMARY OF THE INVENTION

It is accordingly an object of the present invention to provide an endoscopic image filing system that is capable of improving the accuracy of an endoscopy diagnosis by recording the condition of the patient in the endoscopic examination along with endoscopic images, referring to the patient's condition while playing back images, or recording the patient's condition in an examination report.

The present invention provides an endoscopic image filing system for recording information on the condition of an examined body in an endoscopic examination having the features recited in claim 1. Preferred features of the invention are recited in the dependent claims. The present invention also provides a computer program product for managing an endoscopic image filing system, as defined in claim 6.

The endoscopic image filing system of the present invention is constituted comprising: a first communication section for capturing an endoscopic image signal from an endoscope apparatus that obtains an endoscopic image signal by observing an examined body by means of an endoscope; a second communication section for capturing biological information from a measuring apparatus that obtains the biological information by measuring the examined body being observed by the endoscope; a storage section for storing data; and a registration section for registering, in the storage section, the biological information and the endoscopic image signal thus captured as examination data on the examined body, such that the biological information and the endoscopic image signal are related on the basis of pre-registered information identifying the examined body. The registration section is adapted to register the biological information at predetermined periodic intervals, which able to be set for the examination.

Other characteristic features and benefits of the present invention will be made sufficiently apparent by means of the following description.

### BRIEF DESCRIPTION OF THE DRAWINGS

Figs. 1 to 12 relate to an embodiment of the present invention.
Fig. 1 is a constitutional view of the constitution of the endoscopic image filing system;
Fig. 2 is a constitutional view of the constitution of the endoscope system in Fig. 1;
Fig. 3 is a constitutional view of the constitution of the image filing apparatus in Fig. 1;
Fig. 4 is a constitutional view of the constitution of the patient monitoring apparatus in Fig. 1;
Fig. 5 is an explanatory diagram that provides an overview of the configuration of the windows of the image filing apparatus in Fig. 3;
Fig. 6 is a flowchart to explain an example of the overall flow of the operation of the image filing apparatus in Fig. 3;
Fig. 7 is a first flowchart to explain the operation of setting various information related to patient biological information by means of the image filing apparatus in Fig. 3;
Fig. 8 is a second flowchart to explain the operation of setting various information related to patient biological information by means of the image filing apparatus in Fig. 3;
Fig. 9 shows a template which has been edited using the template editing window by means of the image filing apparatus in Fig. 3;
Fig. 10 is a flowchart to explain the operation of an endoscopic examination by means of the endoscopic image filing system in Fig. 1;
Fig. 11 shows an examination performance window that is developed in the process of Fig. 10; and
Fig. 12 shows a patient biological information window for referring to patient biological information by means of the image filing apparatus in Fig. 3.

### DESCRIPTION OF THE PREFERRED EMBODIMENTS

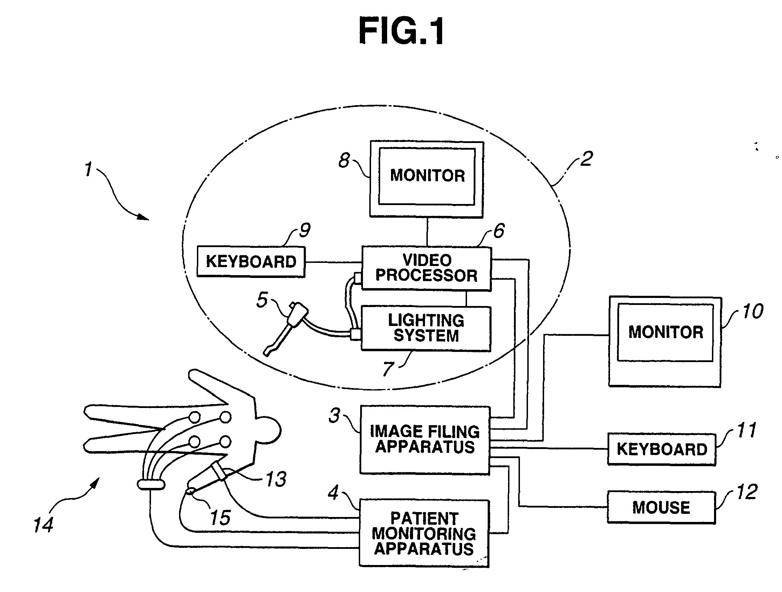

As shown in Fig. 1, the endoscopic image filing system 1 of the present embodiment is principally constituted by an endoscope system 2 for performing an examination by means of endoscopic images; an image filing apparatus 3, which is connected to the endoscope system 2 and is for recording desired endoscopic images; and a patient monitoring apparatus 4 for monitoring biological information such as the patient's blood pressure and pulse, and an electrocardiogram.

The endoscope system 2 is constituted by: an endoscope 5, which is inserted into a body cavity to pick up images of an examination site; a video processor 6 for processing image pickup signals from the endoscope 5 to generate endoscopic images; a light source 7 for supplying illuminating light to the endoscope 5; and a monitor 8 for displaying endoscopic images that are generated by the video processor 6.

A keyboard 9 for inputting data is connected to the video processor 6. A monitor 10 for displaying endoscopic images and data, a keyboard 11 for inputting data, and a mouse 12 are connected to the image filing apparatus 8.

Connected to the patient monitoring apparatus 4 are: a cuff 13 for measuring the patient's blood pressure; an electrode section 14 constituted by a plurality of electrodes and cables for measuring a patient electrocardiogram; and a probe 15, which comprises a red light light-emitting portion and light-receiving portion and which is for measuring the patient's arterial blood oxygen concentration and pulse.

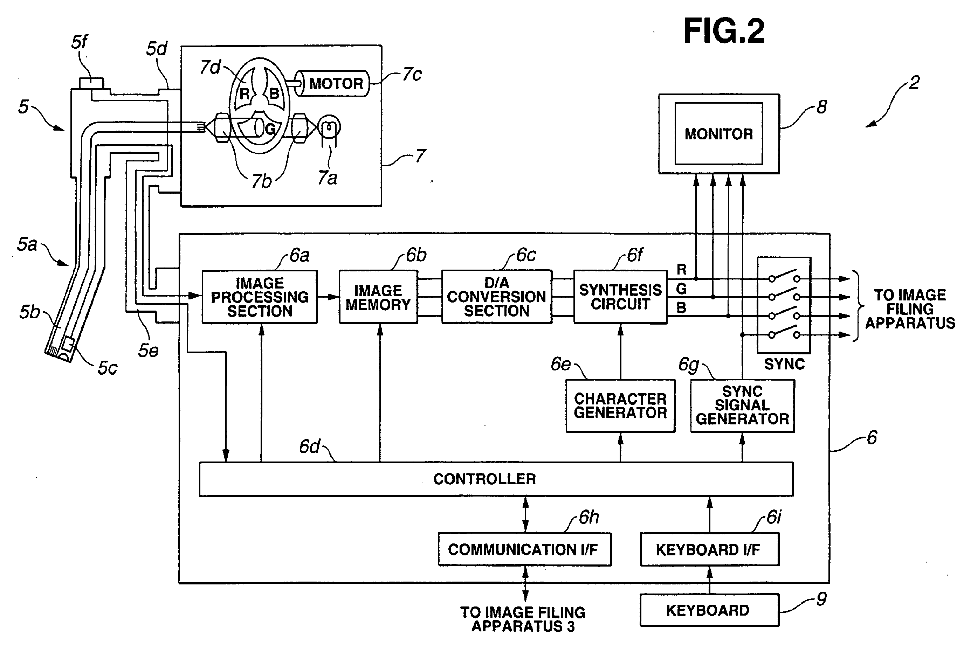

As shown in Fig. 2, the endoscope 5 comprises an insert portion 5a, which is formed to be flexible and long and thin. The insert portion 5a is provided with a light guide 5b for transmitting illuminating light, which is for illuminating an examination site, from the base side of the endoscope 5 to the tip of the insert portion 5a thereof, the base side of the light guide 5b being connected to the light source 7.

The tip of the insert portion 5a is provided with an imaging apparatus 5c for picking up images of an examination site and comprising a CCD or similar, which is a solid-state image sensor, for example. The endoscope 5 is connected by means of a connector 5d to the light source 7 and is also connected to the video processor 6 by means of a universal cable 5e. The endoscope 5 further comprises, at the base side thereof, a release switch 5f for inputting an instruction to record an image.

Light emitting means 7a, such as a lamp, for emitting illuminating light are provided in the light source 7. The illuminating light emitted by the light emitting means 7a is condensed by a condensing optical system 7b and enters the light guide 5b. A rotating plate, which is rotated by a motor 7c and has three optical filters 7d attached thereto which are red, green and blue in color respectively (written as R, G, B respectively in Fig. 2), is disposed at a midway point of the light path of the condensing optical system 7b, whereby the color of the illuminating light illuminating the examination site can be switched using time division.

An image signal of the examination site which is picked up by imaging apparatus 5c is inputted to an image processing section 6a inside the video processor 6, and image processing such as color highlighting for example is carried out, such images being temporarily stored in sequence in an image memory 6b. Image data stored in the image memory 6b is inputted to a D/A conversion section 6c where this data is D/A converted, and the D/A convers ion section 6c outputs an RGB format image signal. The image processing section 6a, image memory 6b, and the like, operate under the control of a controller 6d which constitutes control means for controlling components in the video processor 6. The image signal which is outputted from the D/A conversion section 6c is synthesized by a synthesis circuit 6f with an image signal outputted from a character generator 6e described hereinafter. The RGB format image signal which is output from the synthesis circuit 6f is inputted to the monitor 8 to cause an image of the examination site to be displayed on the monitor 8. At such time, a sync signal (written as SYNC in Fig. 2), which is supplied to the monitor 8, is generated by a sync signal generator 6g that is controlled by the controller 6d.

Character information is inputted by the controller 6d to the character generator 6e such that the character generator 6e is then able to output the character information thus inputted following conversion thereof into an image signal. In other words, character information can then be synthesized and displayed as an image of the examination site in a window of the monitor 8, and it is possible to transmit various messages to the user of the endoscope system 2.

The image signal which is output from the synthesis circuit 6f is not only output to the monitor 8 but rather is also output to the image filing apparatus 3 so as to also be displayable by the image filing apparatus 3.

Further, the controller 6d is also constituted so as to be capable of transmitting and receiving information to and from the image filing apparatus 3 via a communication interface 6h of the commonly known RS-232C system for example. In addition, the controller 6d is connected to the release switch 5f and is thus capable of detecting the state of the release switch 5f.

A keyboard interface 6i is for detecting inputs from the keyboard 9.

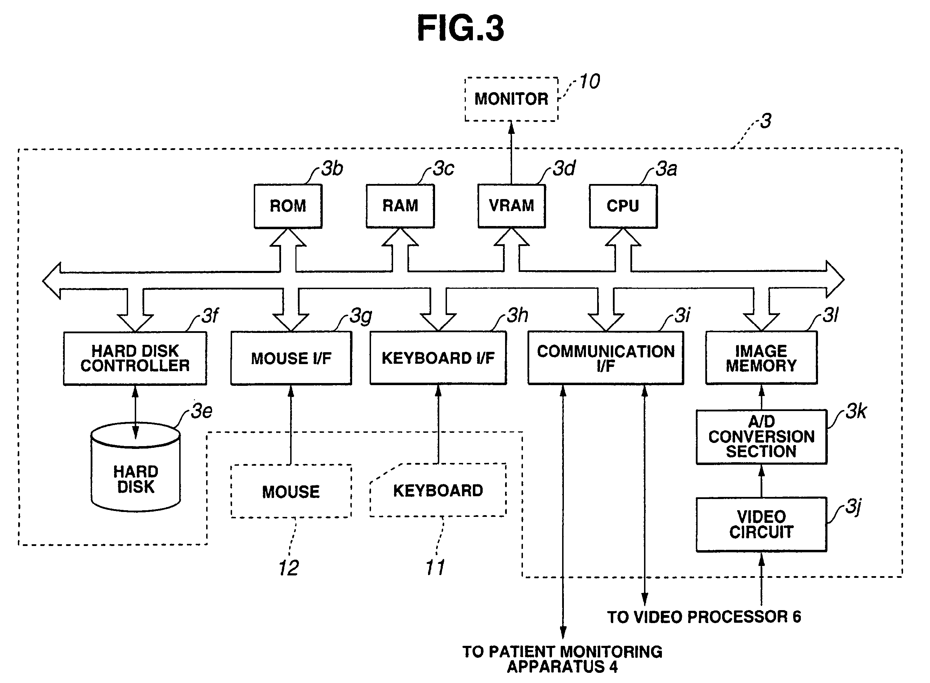

As shown in Fig. 3, the image filing apparatus 3 possesses the function of a storage section, a registration section, a report creation section, and a selecting section, and the like, and comprises: a CPU 3a, which constitutes the principal control unit for controlling the components of the image filing apparatus 3; a ROM 3b, in which programs which cause the CPU 3a to operate, or similar, are stored; a RAM 3c, which is used as the work area of the CPU 3a and as a buffer for various data; a VRAM 3d for temporarily storing image data which is output to the monitor 10; a hard disk 3e for storing image data and various data; a hard disk controller 3f for reading and writing data to and from the hard disk 3e; a mouse interface 3g for detecting inputs via the mouse 12; a keyboard interface 3h for detecting inputs via the keyboard 11; a communication interface 3i which uses the commonly known RS-232C system for example and is a second communication section for transmitting and receiving various data to and from the video processor 6 and the patient monitoring apparatus 4; a video circuit 3j, which is a first communication section constituting an interface circuit for inputting an image signal which is outputted from the video processor 6; an A/D conversion section 3k for A/D converting an image signal which is inputted by the video circuit 3j; and an image memory 31 for temporarily storing image data which is output from the A/D conversion section 3k.

By means of the aforementioned components, the image filing apparatus 3 is capable of displaying image data obtained by the endoscope system 2 on the monitor 10, storing this image data on the hard disk 3e, and so forth.

Also, by obtaining the state of the release switch 5f, the image filing apparatus 3 is also capable of dividing up processing by the CPU 3a, to permit the recording of images for example.

In accordance with the various windows displayed on the monitor 10, the image filing apparatus 3 executes processing such that the operator inputs data and instructions via the keyboard 11, mouse 12, and the like, and such that the CPU 3a controls each component in accordance with inputted data and/or instructions. That is, the image filing apparatus 3 is thus capable of executing various processing in accordance with the flow of the windows displayed on the monitor 10.

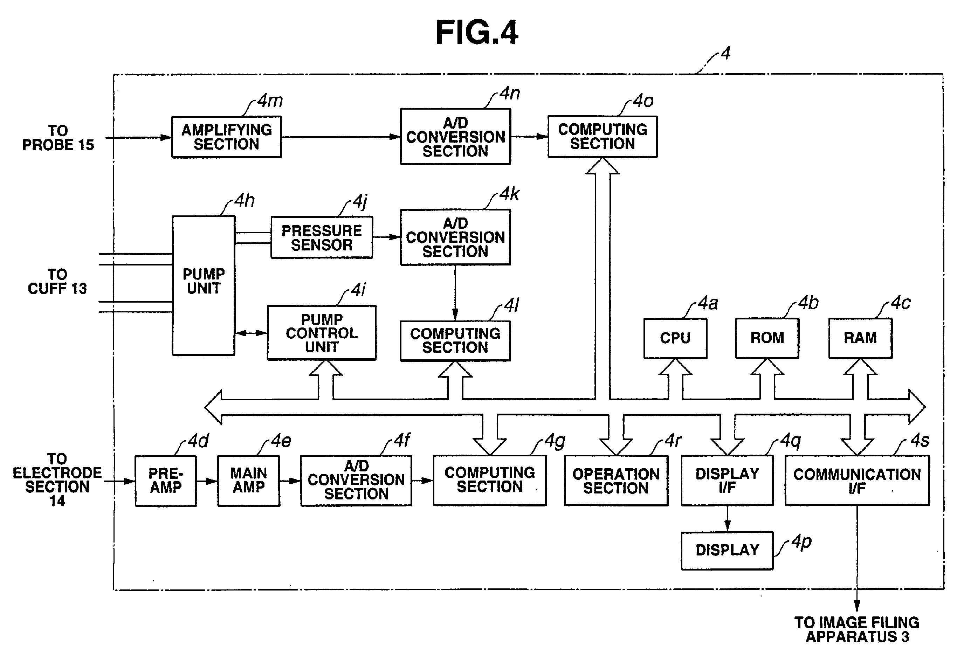

As shown in Fig. 4, the patient monitoring apparatus 4 comprises: a CPU 4a, which constitutes the principal control unit for controlling the components of the patient monitoring apparatus 4; a ROM 4b, in which programs which cause the CPU 4a to operate, or similar, are stored; a RAM 4c, which is used as the work area of the CPU 4a and as a buffer for various data; a pre-amp 4d, which is electrically isolated from the power supply (not illustrated) of the patient monitoring apparatus 4 and is for amplifying weak electrical signals detected by the electrode section 14 attached to the patient; a main amp 4e, which further amplifies electrical signals amplified by the pre-amp 4d to a magnitude permitting processing; an A/D conversion section 4f for A/D converting electrical signals amplified by the main amp 4e; a computing section 4g for computing electrical signals inputted from the A/D conversion section 4f to thereby calculate various forms of electrocardiogram, the patient's pulse, and the like; a pump unit 4h constituted by a pump (not illustrated) for supplying air to the cuff 13, and an air valve (not illustrated); a pump control unit 4i for controlling the pump unit 4h; a pressure sensor unit 4j for measuring the pressure acting on the cuff 13 via the pump unit 4h; an A/D conversion section 4k for A/D converting numerical values inputted from the pressure sensor unit 4j; a computing section 41 for calculating values such as the maximum blood pressure and the minimum blood pressure from numerical values inputted from the A/D conversion section 4k; an amplifying section 4m for amplifying signals transmitted by the light-receiving section (not illustrated) causing the light-emitting section (not illustrated) of the probe 15 to emit light; an A/D conversion section 4n for A/D converting signals transmitted by the amplifying section 4m; a computing section 4o for computing signals transmitted by the A/D conversion section 4n to thereby calculate the arterial blood oxygen concentration, heart rate, and the like; a display 4p for displaying data for an electrocardiogram, the heart rate, or the like, calculated by the computing section 4g; data for the blood pressure and heart rate, and the like, calculated by the computing section 41; and data for the arterial blood oxygen concentration, heart rate, and the like, calculated by the computing section 4o; a display interface 4q for transmitting data to the display 4p; an operation section 4r permitting the operator to issue instructions; and a communication interface 4s of the commonly known RS-232C system for example, for transmitting and receiving various data to and from the image filing apparatus 3.

By means of the aforementioned components, the patient monitoring apparatus 4 is capable of measuring the patient's pulse, an electrocardiogram, and the like, as a result of the electrode section 14 detecting weak electrical signals generated in accordance with contractions of the myocardium and also by amplifying and computing detected electrical signals. The measurement data can be stored in the RAM 4c and displayed on the display 4p in real time in the format desired by the operator, and numerical value data for a pulse or the like can be transmitted to the image filing apparatus 3.

Furthermore, the patient monitoring apparatus 4 is capable of detecting pulse waves and pressures thereof in accordance with pulsations produced by supplying air to the cuff 13 attached to the patient to thereby measure the patient's blood pressure (maximum blood pressure and minimum blood pressure), and of displaying the patient's blood pressure on the display 4p and transmitting numerical values for the patient's blood pressure to the image filing apparatus 3. These blood pressure measurements cannot be measured as serial values but the user can, in accordance with requirements, make measurements with respect to optional timing by operating the operation section 4r, or can also repeat the measurements periodically in accordance with a program. In the latter case, the measurement interval is set by the operator by operating the operation section 4r. These measured values are stored in the RAM 4c until the next measurements are made in accordance with optional timing or a program.

The patient monitoring apparatus 4 is also capable of calculating the arterial blood oxygen concentration (SpO₂) and heart rate by calculating the difference in the transmittance of red light of two different wavelengths detected by the probe 15 attached to the patient. This measurement data can be stored in the RAM 4c and displayed on the display 4p in real time and numerical values of this data can be transmitted to the image filing apparatus 3.

The patient monitoring apparatus 4 of this constitution possesses the functions of a boundary value setting section, a comparing section, and a notifying section, and the like, and is capable of setting boundary values for each measured value and of providing a warning by means of the display 4p, or the like, when measurement results exceed boundary values. For example, when a user uses the operation section 4r to input maximum and minimum heart rates, a minimum value for the minimum blood pressure, a maximum value for the maximum blood pressure, and a minimum value for the arterial blood oxygen concentration, and the like, a warning can be displayed on the display 4p and a warning sound can be emitted by a speaker (not illustrated) in cases where measured values exceed these set values.

The patient monitoring apparatus 4 is also capable of transmitting data for the patient's electrocardiogram, pulse, blood pressure, arterial blood oxygen concentration and the like, by means of a communication system determined by the manufacturer, via the communication interface 4s to an image filing apparatus or other external device.

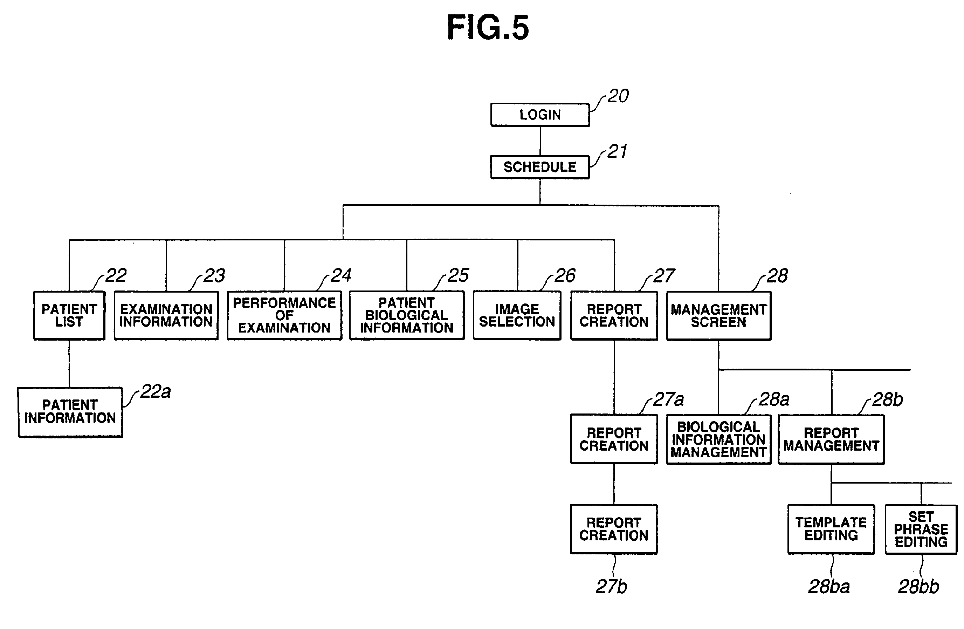

An overview of the configuration of the windows of the image filing apparatus 3 will now be provided using Fig. 5.

First, upon startup of the image filing apparatus 3, a login window 20 for user authentication is displayed on the monitor 10. Following user authentication by means of the login window 20, a schedule window 21, which is for displaying a list of examination schedules or similar, is displayed on the monitor 10.

A patient list window 22 for displaying a list of patient information can be called from the schedule window 21. A patient information window 22a for newly registering patient information or editing patient information which has already been registered can be called from the patient list window 22.

Further, an examination information window 23 for reserving an examination by newly registering examination information or editing examination information which has already been registered can be called from the schedule window 21.

An examination performance window 24 for performing an examination by means of a connection with the endoscope system 2 and capturing images from the endoscope system 2 can also be called from the schedule window 21.

A patient biological information window 25 for referring to and editing captured biological information in the course of an endoscopic examination can be also called from the schedule window 21.

An image selection window 26 for selecting images to be attached to an examination report that is created, from among captured images, can also be called from the schedule window 21.

A report creation window 27, which constitutes one of the windows for creating an examination report, can be called from the schedule window 21, and a transition can also be made from the report creation window 27 to report creation windows 27a, 27b which have different functions.

Further, a management window 28 for setting the various operations of the image filing apparatus 3 can be called from the schedule window 21. A biological information management window 28a for setting operations related to biological information (described hereinafter), and a report management window 28b for setting operations related to report creation, for example, or the like, can be called from the management window 28. In addition, a template editing window 28ba for editing report templates, and a set phrase editing window 28bb for editing set phrases used for report creation, for example, or similar, can be called from the report management window 28b.

Next, the operation of the present embodiment having such a constitution will be described.

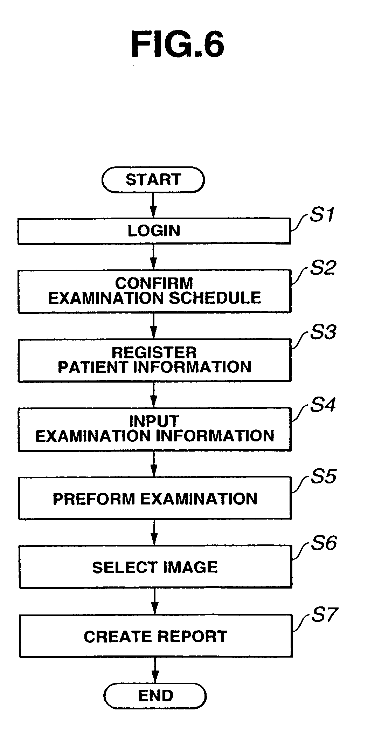

An example of the overall flow of the operation of the image filing apparatus 3 will be described using Fig. 6.

First, upon startup of the image filing apparatus 3, the login window 20 is displayed in step S1, and operator authentication is performed here. When the operator logs in following authentication, the schedule window 21 is displayed in step S2, whereupon an examination schedule is confirmed.

Thereafter, when the patient to undergo the examination is a new patient, in step S3, patient information is registered after calling the patient list window 22 and the patient information window 22a. Next, in step S4, a new examination reservation is inputted after calling the examination information window 23.

Next, in step S5, the examination performance window 24 is called and an examination is performed using the endoscope system 2, which is connected to the image filing apparatus 3. Images obtained by the endoscope system 2 are stored in the image filing apparatus 3. After the examination has ended, in step S6, the image selection window 26 is called and images to be used in an examination report that is created are selected from among images obtained while the examination was performed. In step S7, the report creation windows 27, 27a, 27b are called and an examination report is created. Further, according to requirements, the patient biological information window 25 can be called with respect to optional timing after the examination has ended, and patient biological information captured in the course of the examination can be referenced. The above description constitutes an example of the overall flow of operation.

Independently of the overall flow of operation, it is possible, according to requirements, to call the management window 28 with respect to optional timing to perform setting for the various operations of the image filing apparatus 3.

Next, the operation for performing setting of the various operations of the image filing apparatus 3 in the management window 28 will be described. The management window 28 is used, for example, to register and edit information on patients and examiners which is inputted as examination information, to perform setting of required items of patient information and examination information, and so forth, setting for various information related to report creation, and setting for various information related to patient biological information.

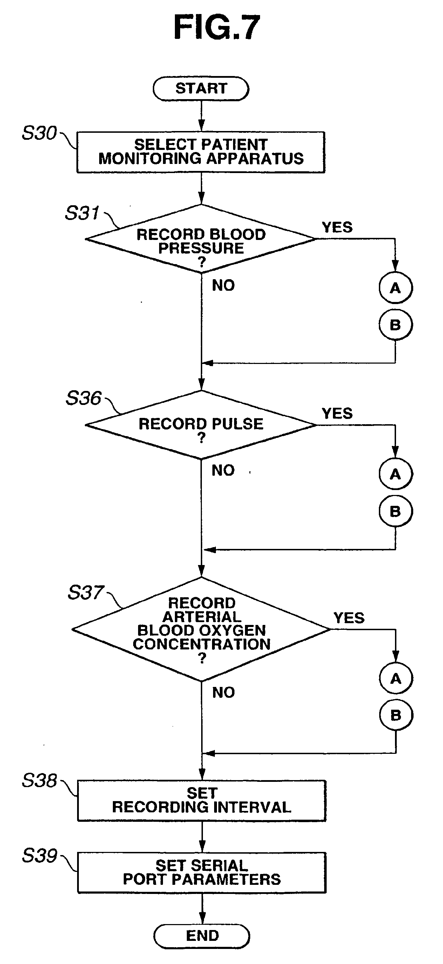

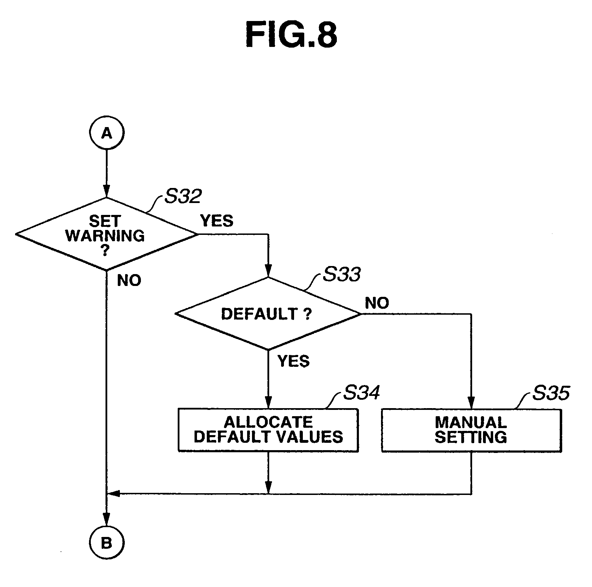

Of the operations of the management window 28, a description will first be provided for the operation of setting various information related to patient biological information, using Figs. 7 and 8. The biological information management window 28a is called from the management window 28.

First, the patient monitoring apparatus 4 is selected for use in step S30. Next, in step S31, a choice is made of whether or not to make a blood pressure recording. When a blood pressure recording is not made, step S36 follows. When a blood pressure recording is made, step S32 follows and a choice is made of whether or not to set maximum and minimum blood pressure values at which a warning is to be provided. When such values are to be set, step S33 follows and a choice is made of whether or not to allocate default maximum and minimum blood pressure values as the set values. When default values are allocated, step S34 follows and the default maximum and minimum blood pressure values are allocated as the set values, and processing proceeds to step S36. A minimum blood pressure of 40 mmHg and a maximum blood pressure of 180 mmHg, for example, can be set as default values. When default values are not allocated, step S35 follows, and the maximum and minimum blood pressure values are set manually, and processing proceeds to step S36. When maximum and minimum blood pressure values at which a warning is to be provided are not set, processing proceeds to step S36 without any setting being made.

Next, in step S36, a choice is made of whether or not to make a pulse rate recording. When a pulse rate recording is not made, step S37 follows. When a pulse rate recording is made, step S32 follows, whereupon a choice is made of whether or not to set maximum and minimum pulse rates at which a warning is to be provided. When such pulse rates are set, step S33 follows and a choice is made of whether or not to allocate default maximum and minimum pulse rates as set values. When default values are allocated, step S34 follows and default maximum and minimum pulse rates are allocated as set values, and processing proceeds to step S37. A minimum pulse rate of 40 beats per minute and a maximum pulse rate of 180 beats per minute, for example, can be set as default values. When default values are not allocated, step S35 follows, and the maximum and minimum pulse rates are set manually, and processing proceeds to step S37. When maximum and minimum pulse rates at which a warning is to be provided are not set, processing proceeds to step S37 without any setting being made.

Next, in step S37, a choice is made of whether or not to make an arterial blood oxygen concentration recording. When an arterial blood oxygen concentration recording is not made, step S38 follows. When an arterial blood oxygen concentration recording is made, step S32 follows, whereupon a choice is made of whether or not to set maximum and minimum an arterial blood oxygen concentrations at which a warning is to be provided. When such arterial blood oxygen concentrations are set, step S33 follows and a choice is made of whether or not to allocate default maximum and minimum arterial blood oxygen concentrations as set values. When default values are allocated, step S34 follows and default maximum and minimum arterial blood oxygen concentrations are allocated as set values, and processing proceeds to step S38. A minimum arterial blood oxygen concentration of 85 (%) and a maximum arterial blood oxygen concentration of 100 (%), for example, can be set as default values. When default values are not allocated, step S35 follows, and the maximum and minimum arterial blood oxygen concentrations are set manually, and processing proceeds to step S38. When maximum and minimum arterial blood oxygen concentrations at which a warning is to be provided are not set, processing proceeds to step S38 without any setting being made.

Next, an interval for recording biological information is set in step S38. Thereafter, in step S39, the serial port parameters are set. Here, for example, the port number to which the patient monitoring apparatus 4 is connected, the data bits, the stop bit, the parity, and the like, are set. When setting is complete, the setting of various information relating to patient biological information ends.

Next, among the operations of the management windows 28, a description is provided for the operation of setting various information related to report creation. The report management window 28b, which is called from the management window 28, permits the setting of various conditions and so forth related to the editing of set phrases and medical terms or similar used for opinions appearing in reports, the editing of report templates, and the editing of reports.

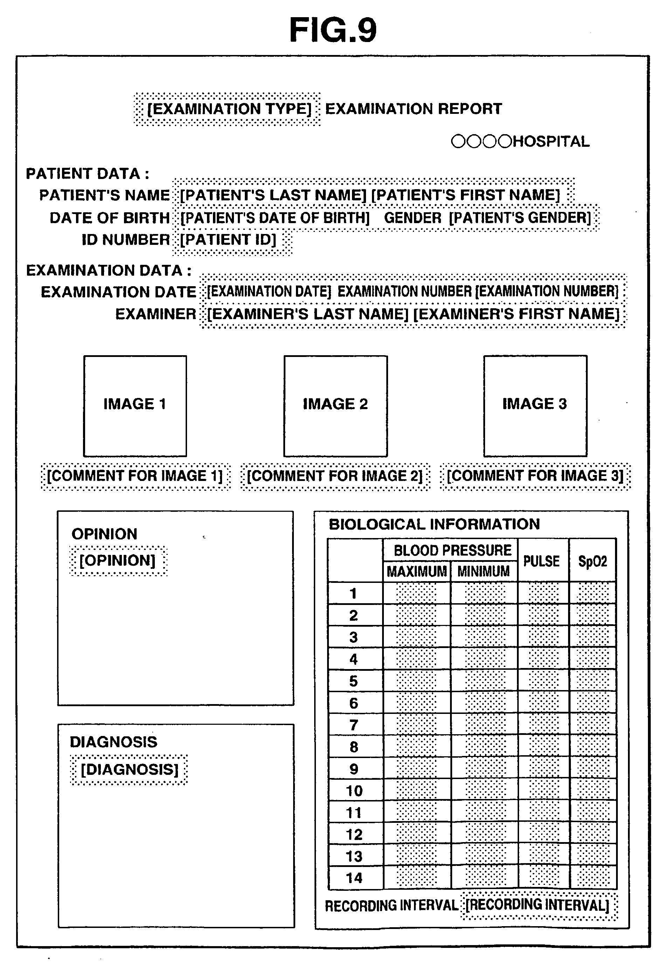

Here, a description will be provided using Fig. 9 for the operation of editing a report template, which is performed using the template editing window 28ba.

Patient information and examination information registered in steps S3 and S4 of Fig. 6 are registered in a database, and this data can be reflected in a report. The operator enters optional text in the template editing window 28ba, and inserts information registered in the database, to thereby edit a template. An opinion and diagnostic information entered in the report creation window 27, patient biological information registered during an examination by means of an operation described hereinafter, and images selected by means of the image selection window 26 can also be reflected in a report. Fig. 9 shows an example of a template which has been edited using the template editing window 28ba. In Fig. 9, information registered in the database is allocated as half-tone dot meshed text.

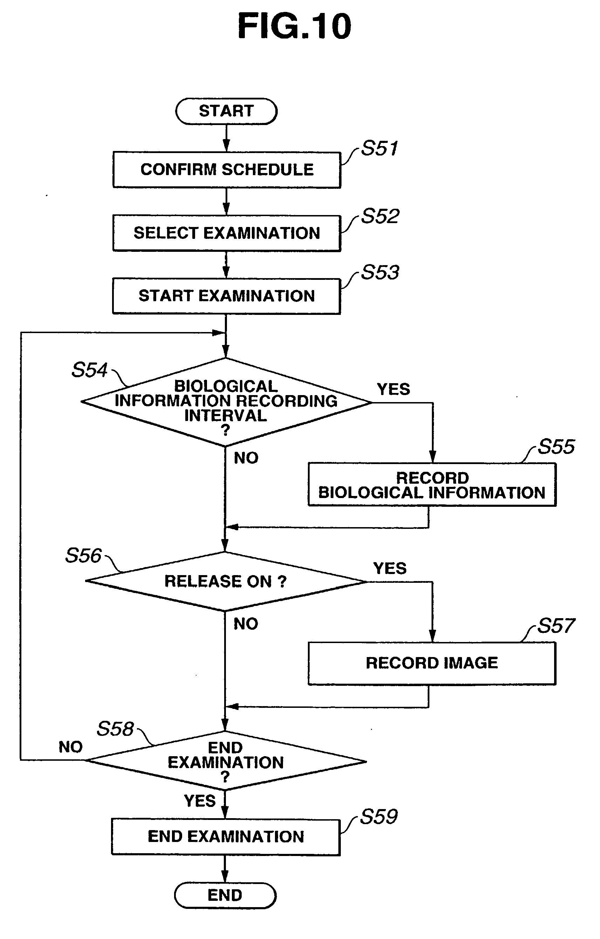

Next, a description is provided for the operation of an actual endoscopic examination, using Fig. 10. First, in step S51, the schedule window 21 opens, and examinations which have been reserved for that day are confirmed, and, in step S52, the examination in question is selected, and the examination performance window 24 opens.

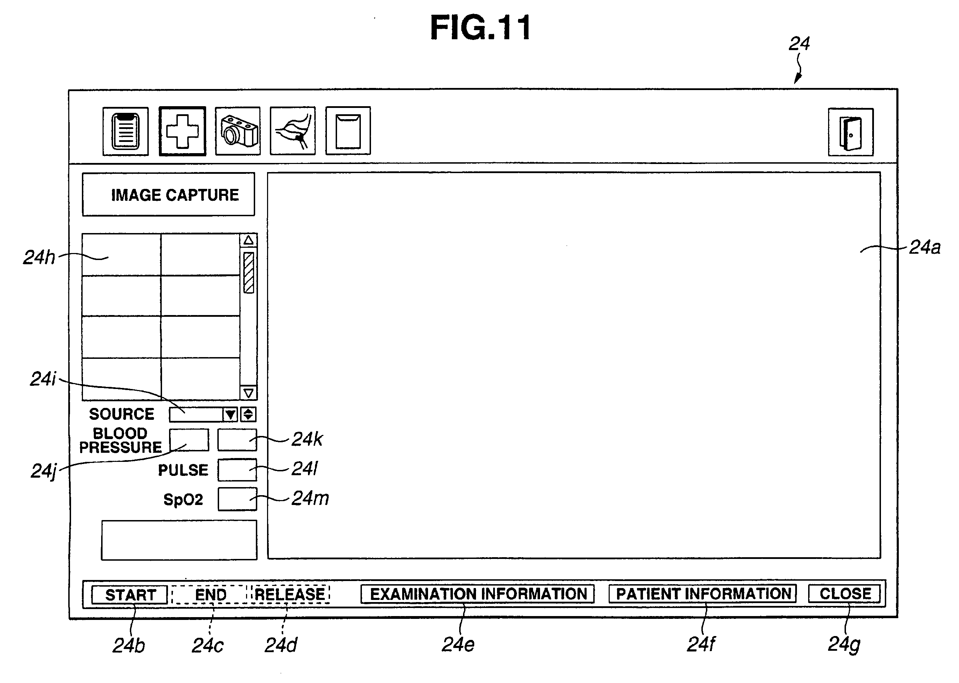

As shown in Fig. 11, the examination performance window 24 has disposed therein: a display area 24a for displaying in real time endoscopic images picked up by the video processor 6; a Start button 24b for starting the examination; an End button 24c for ending the examination; a Release button 24d for performing image recording; an examination information button 24e for displaying examination information of an examination being performed; a patient information button 24f for displaying patient information of a patient being examined; and a Close button 24g for closing the examination performance window 24. Further disposed in the examination performance window 24 are: an index image area 24h for displaying recorded images in reduced form; a source field 24i for displaying a variety of connected video processors; a maximum blood pressure field 24j and a minimum blood pressure field 24k for displaying maximum and minimum blood pressures respectively recorded by means of an operation described hereinafter; a pulse field 241 for displaying the patient's pulse; and an arterial blood oxygen concentration (SpO₂) field 24m for displaying arterial blood oxygen concentration.

In step S53, the examination is started by pressing an examination start button (not illustrated) of the keyboard 9, or the start button 24b. The examination starts and, in step S54, if the time of the interval for recording biological information (set by means of the operation described earlier) elapses after biological information recording is performed at the start or end of the examination, biological information recording is performed in step S55. In this recording operation, the image filing apparatus 3 accesses the communication interface 4s of the patient monitoring apparatus 4 via the communication interface 3i, and biological information held in the RAM 4c is read in accordance with the serial port parameters and recorded on the hard disk 3e along with the actual time. Each item of biological information is registered in the database so as to be related with registered examination information. Here, each type of measured biological information is displayed in the maximum blood pressure field 24j, the minimum blood pressure field 24k, the pulse field 241, and the arterial blood oxygen concentration field 24m respectively. When the time of the recording interval has not elapsed, processing proceeds to step S56.

When it is detected in step S56 that the release switch 5f of the endoscope 5, or the release button 24d is depressed, step S57 follows and image recording is performed. An image signal inputted from the video processor 6 via the video circuit 3j is A/D converted by the A/D conversion section 3k and stored in the image memory 31. When the release switch 5f or the release button 24d is depressed, this A/D converted image signal is recorded on the hard disk 3e. At such time, each image is registered in the database so as to be related with registered examination information. When image recording is complete, step S58 follows, and, when it is not detected that the examination end button (not illustrated) of the keyboard 9, or the end button 24c is depressed, processing returns to step S54. When neither the release switch 5f nor the release button 24d is depressed and it is detected in step S58 that the examination end button or the end button 24c is depressed, processing proceeds to step S59 and the examination ends.

That is, one itemof examination information is constituted by examination information comprising patient information registered in an examination reservation, and image information and biological information recorded during the examination.

In cases where warning values are set, when measured values that exceed the warning values are recorded in step S55, these measured values are recorded with a warning flag attached thereto. This warning flag can be shown, for example, by changing the color of a measured value or by means of half-tone dot meshing processing, when displaying biological information recorded using the patient biological information window 25.

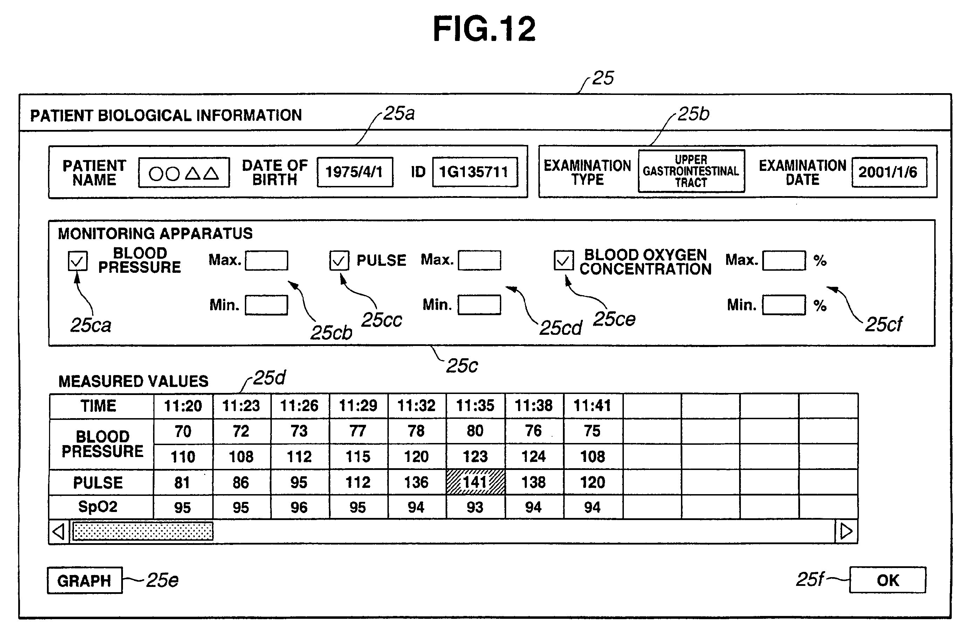

Next, a description is provided for the operation of referencing patient biological information which has been recorded in this manner. An examination which has been performed is selected in the schedule window 21, and when the patient biological information window 25 is called, the patient biological information window 25 opens as shown in Fig. 12. The patient biological information window 25 comprises: a patient information area 25a for displaying patient information entered in the patient informationwindow22a; an examination information area 25b for displaying examination information entered in the examination information window 23; a monitoring apparatus information area 25c for displaying information entered in the biological information management window 28b; a measured value area 25d for displaying patient biological information recorded in the examination performance operation described hereinabove; a graph button 25e for converting information in the measured value area 25d into a graph; and an OK button 25f for closing the patient biological information window 25.

The monitoring apparatus information area 25c comprises:
a blood pressure check box 25ca; blood pressure maximum value and minimum value display sections 25cb; a pulse check box 25cc; pulse maximum value and minimum value display sections 25cd; an arterial blood oxygen concentration check box 25ce; and
arterial blood oxygen concentration maximum value and minimum value display sections 25cf.

When, in the operation of setting various information related to patient biological information described earlier, setting is performed to make a blood pressure measurement, the blood pressure check box 25ca is checked automatically and set blood pressure maximum and minimum values are displayed in the blood pressure maximum and minimum value display sections 25cb respectively. When setting is performed to make a pulse measurement, the pulse check box 25cc is checked automatically and set pulse maximum and minimum values are displayed in the pulse maximum and minimum value display sections 25cd respectively.

When setting is performed to make an arterial blood oxygen concentration measurement, the arterial blood oxygen concentration check box 25ce is checked automatically and set arterial blood oxygen concentration maximum and minimum values are displayed in the arterial blood oxygen concentration maximum and minimum value display sections 25cf respectively. Patient biological information which is recorded while an examination is performed is displayed chronologically in the measured value area 25d. Among the measured values, values recorded with an attached warning flag at the time of image recording, which are higher than a set maximum value or lower than a set minimum value are displayed in a color different from that used for normal values or are displayed with half-tone dot meshing as shown in Fig. 11.

Clicking on the graph button 25e causes the values displayed in the measured value area 25d to be displayed as a line graph or the like. Pushing the OK button 25f causes the patient biological information window 25 to close. Further, data displayed in the patient biological information window 25 is preset data or data recorded during an examination, and cannot be changed after the examination has ended.

A description follows for the operation of creating an examination report using image information and biological information thus recorded.

The schedule window 21 is used to select an examination which has been performed, and to then call the image selection window 26 where endoscopic images recorded during an examination are confirmed and images that are to be attached to a report are selected. If required, comments are appended to selected images.

Processing then moves to the report creation window 27 in which examination information that comprises patient information inputted at the time of a reservation, endoscopic images selected using the image selection window 26, and biological information are already inputted. Here, opinions and supplementary information are entered by the examiner and, when an edited template is called in the template editing window 28ba, the above-described patient information, examination information, endoscopic images, and biological information are respectively allocated to locations allocated for information registered in a template database, to thereby complete one report. In this report, with respect to biological information recorded while an examination is performed, values which are higher than a set maximum value or lower than a set minimum value are displayed in a color different from that used for normal values or are displayed with half-tone dot meshing, similarly to the patient biological information window 25.

Other than the restrictions placed by the attached claims, the present invention is not limited to or by specific embodiments.

## Claims

1. Endoscopic image filing system for recording information on the condition of an examined body in an endoscopic examination, comprising:
a first communication section configured to connect to an endoscope apparatus (2) and capture from the endoscope apparatus (2) an endoscopic image signal obtained by observing an examined body by means of an endoscope (5);
a second communication section configured to connect to a measuring apparatus (4) and capture from the measuring apparatus (4) biological information obtained by measuring the examined body being observed by the endoscope (5);
a storage section (3e) for storing data; and
a registration section configured to register, in the storage section (3e), the biological information and the endoscopic image signal thus captured as examination data on the examined body, to thereby relate the biological information and the endoscopic image signal on the basis of pre-registered information identifying the examined body;
**characterized in that** the registration section is adapted to register the biological information at predetermined periodic intervals, which are settable for the examination.

2. The system of claim 1, further comprising a report creation section configured to create a report that comprises the biological information and endoscopic image data based on the endoscopic image signal, on the basis of the examination data registered in the storage section (3e).

3. The system of claim 1 or 2, further comprising:
a boundary value setting section configured to set boundary values for the captured biological information;
a comparing section configured to compare the boundary values and values of the captured biological information; and
a notifying section configured to provide notification in accordance with a comparison result of the comparing section.

4. The system of claim 1 or 2, further comprising a selecting section configured to select at least one of blood pressure, pulse, arterial blood oxygen concentration, and an electrocardiogram as the biological information, wherein the registration section is further configured to register biological information selected by the selecting section in the storage section (3e) such that the biological information is related with the endoscopic image signal.

5. The system of claim 1, further comprising an instructing section (24d) configured to instruct that the endoscopic image signal be captured, wherein the registration section is configured to register, in the storage section (3e), the biological information and the endoscopic image signal as captured in accordance with an instruction of the instructing section (24d).

6. A computer program product for managing an endoscopic image filing system for recording information on the condition of an examined body in an endoscopic examination and endoscopic image information, comprising software code portions for performing the following steps in a system according to claim 1:
a first communication step (S57) of capturing from an endoscope apparatus (2) an endoscopic image signal obtained during examination of an examined body by means of an endoscope (5);
a second communication step (S55) of capturing from a measuring apparatus (4) biological information obtained during measurement of the examined body observed by the endoscope (5); and
a registration step of registering, in a storage section (3e), the biological information and the endoscopic image signal thus captured as examination data on the examined body such that the biological information and the endoscopic image signal are related on the basis of pre-registered information identifying the examined body;
**characterized in that** the registration step registers the biological information at predetermined periodic integrals which are settable for the examination.

7. The computer program product of claim 6, further comprising software code portions for performing a report creation step (S7) of creating a report that comprises the biological information and endoscopic image data based on the endoscopic image signal, on the basis of the examination data registered in the storage section (3e).

8. The computer program product of claim 6 or 7, further comprising software code portions for performing:
a boundary value setting step (S34, S35) of setting boundary values for the captured biological information;
a comparing step of comparing the boundary values and values of the captured biological information; and
a notifying step of providing notification in accordance with a comparison result of the comparing step.

9. The computer program product of claim 6 or 7, further comprising software code portions for performing a selecting step (S30) of selecting at least one of blood pressure, pulse, arterial blood oxygen concentration, and an electrocardiogram as the biological information, wherein the registration step registers biological information selected by the selecting step in the storage section (3e) such that the biological information is related with the endoscopic image signal.

## Patentansprüche

1. Akquisitionssystem für endoskopische Bilder zur Aufzeichnung von Informationen betreffend den Zustand eines untersuchten Körpers bei einer endoskopischen Untersuchung, aufweisend:
einen ersten Kommunikationsabschnitt, der mit einer Endoskopvorrichtung (2) verbindbar ist und von der Endoskopvorrichtung (2) ein endoskopisches Bildsignal aufzunehmen vermag, das durch Beobachten eines untersuchten Körpers mittels eines Endoskops (5) erhalten wird;
einen zweiten Kommunikationsabschnitt, der mit einer Messvorrichtung (4) verbindbar ist und von der Messvorrichtung (4) eine biologische Information aufzunehmen vermag, die durch Messung am durch das Endoskop (5) beobachteten untersuchten Körper erhalten wird;
einen Speicherabschnitt (3e) zur Datenspeicherung; und
einen Aufzeichnungsabschnitt zum Aufzeichnen der so aufgenommenen biologischen Information und des endoskopischen Bildsignals als Untersuchungsdaten an dem untersuchten Körper in dem Speicherabschnitt (3e), um die biologische Information und das endoskopische Bildsignal auf der Grundlage einer vorab aufgezeichneten Information , die den untersuchten Körper identifiziert, zuzuordnen,
**dadurch gekennzeichnet, dass** der Aufzeichnungsabschnitt die biologische Information in bestimmten periodischen Intervallen aufzuzeichnen vermag, die für die Untersuchung festsetzbar sind.

2. Das System nach Anspruch 1, aufweisend einen Berichtserzeugungsabschnitt, der, basierend auf dem endoskopischen Bildsignal, auf der Grundlage der im Speicherabschnitt (3e) aufgezeichneten Untersuchungsdaten einen Bericht zu erzeugen vermag, der die biologische Information und die endoskopischen Bilddaten aufweist.

3. Das System nach Anspruch 1 oder 2, weiterhin mit:
einem Grenzwertsetzabschnitt, der Grenzwerte für die aufgenommene biologische Information zu setzen vermag;
einem Vergleichsabschnitt, der die Grenzwerte und Werte der aufgenommenen biologischen Information zu vergleichen vermag; und
einen Mitteilungsabschnitt, der eine Mitteilung gemäß dem Vergleichsergebnis des Vergleichsabschnitts zu liefern vermag.

4. Das System nach Anspruch 1 oder 2, weiterhin mit einem Wahlabschnitt, der zumindest entweder Blutdruck oder Puls oder arterielle Blutsauerstoffkonzentration oder ein Elektrokardiogramm als die biologische Information auszuwählen vermag, wobei der Aufzeichnungsabschnitt weiterhin die biologische Information im Speicherabschnitt (3e) aufzuzeichnen vermag, die von dem Wahlabschnitt gewählt wurde, so dass die biologische Information dem endoskopischen Bildsignal zugeordnet ist.

5. Das System nach Anspruch 1, weiterhin mit einem Anweisungsabschnitt (24d), der anzuweisen vermag, dass das endoskopische Bildsignal aufzunehmen ist, wobei der Aufzeichnungsabschnitt in dem Speicherabschnitt (3e) die biologische Information und das endoskopische Bildsignal aufzuzeichnen vermag, wie sie gemäß einer Anweisung vom Anweisungsabschnitt (24d) aufgenommen wurden.

6. Ein Computerprogramm-Produkt zur Verwaltung eines Akquisitionssystems für endoskopische Bilder zur Aufzeichnung von Informationen betreffend den Zustand eines untersuchten Körpers bei einer endoskopischen Untersuchung und einer endoskopischen Bildinformation, aufweisend Software-Codeabschnitte zur Durchführung der folgenden Schritte in einem System nach Anspruch 1:
einen ersten Kommunikationsschritt (S57) der Aufnahme eines endoskopischen Bildsignals von einer Endoskopvorrichtung (2), das während der Untersuchung eines untersuchten Körpers mittels eines Endoskops (5) erhalten wird;
einen zweiten Kommunikationsschritt (S55), zur Aufnahme einer biologischen Information von einer Messvorrichtung (4), die durch Messung am durch das Endoskop (5) beobachteten untersuchten Körper erhalten wird;
einen Speicherabschnitt (3e) zur Datenspeicherung; und
einen Aufzeichnungsschritt des Aufzeichnens der so aufgenommenen biologischen Information und des endoskopischen Bildsignals als Untersuchungsdaten an dem untersuchten Körper in einem Speicherabschnitt (3e), so dass die biologische Information und das endoskopische Bildsignal auf der Grundlage einer vorab aufgezeichneten Information , die den untersuchten Körper identifiziert, zugeordnet werden,
**dadurch gekennzeichnet, dass** der Aufzeichnungsschritt die biologische Information in bestimmten periodischen Intervallen aufzuzeichnen vermag, die für die Untersuchung festsetzbar sind.

7. Das Computerprogramm-Produkt nach Anspruch 6, weiterhin mit Software-Codeabschnitten zur Durchführung eines Berichtserzeugungsschrittes (S7) der Erzeugung eines Berichts, der die biologische Information und die endoskopischen Bilddaten basierend auf dem endoskopischen Bildsignal aufweist, auf der Grundlage der im Speicherabschnitt (3e) aufgezeichneten Untersuchungsdaten.

8. Das Computerprogramm-Produkt nach Anspruch 6 oder 7, weiterhin mit Software-Codeabschnitten zur Durchführung von:
einem Grenzwertsetzschritt (S34, S35) zum Setzen von Grenzwerten für die aufgenommene biologische Information;
einem Vergleichsschritt zum Vergleichen der Grenzwerte und Werte der aufgenommenen biologischen Information; und
einem Mitteilungsschritt zum Liefern einer Mitteilung gemäß dem Vergleichsergebnis des Vergleichsschrittes.

9. Das Computerprogramm-Produkt nach Anspruch 6 oder 7, weiterhin mit Software-Codeabschnitten zur Durchführung eines Wahlschrittes (S30) der Auswahl von zumindest entweder Blutdruck oder Puls oder arterieller Blutsauerstoffkonzentration oder eines Elektrokardiogramms als die biologische Information, wobei der Aufzeichnungsschritt weiterhin die biologische Information, die von dem Wahlschritt gewählt wurde, im Speicherabschnitt (3e) aufzeichnet, so dass die biologische Information dem endoskopischen Bildsignal zugeordnet wird.

## Revendications

1. Système d'acquisition d'images endoscopiques destiné à enregistrer des informations relatives à l'état d'un corps examiné lors d'un examen endoscopique, comprenant :
une première section de communication configurée pour être reliée à un endoscope (2) et pour capturer, à partir de l'endoscope (2), un signal d'image endoscopique obtenu en observant un corps examiné à l'aide d'un endoscope (5) ;
une seconde section de communication configurée pour être reliée à un appareil de mesure (4) et pour capturer, à partir de l'appareil de mesure (4), des informations biologiques obtenues en mesurant le corps examiné observé par l'endoscope (5) ;
une section de stockage (3e) destinée à stocker des données ; et
une section d'enregistrement configurée pour enregistrer, dans la section de stockage (3e), les informations biologiques et le signal d'image endoscopique ainsi capturés en tant que données d'examen sur le corps examiné, de façon à associer ainsi les informations biologiques et le signal d'image endoscopique sur la base d'informations préenregistrées identifiant le corps examiné ;
**caractérisé en ce que** la section d'enregistrement est adaptée pour enregistrer les informations biologiques à des intervalles périodiques prédéterminés, qui peuvent être définis pour l'examen.

2. Système selon la revendication 1, comprenant en outre une section de création de rapports configurée pour créer un rapport qui comprend les informations biologiques et les données d'image endoscopique basées sur le signal d'image endoscopique, sur la base des données d'examen enregistrées dans la section de stockage (3e).

3. Système selon la revendication 1 ou 2, comprenant en outre :
une section de définition de valeur limite configurée pour définir des valeurs limite pour les informations biologiques capturées ;
une section de comparaison configurée pour comparer les valeurs limite et les valeurs des informations biologiques capturées ; et
une section de notification configurée pour fournir une notification selon un résultat de comparaison de la section de comparaison.

4. Système selon la revendication 1 ou 2, comprenant en outre une section de sélection configurée pour sélectionner au moins l'un d'une pression artérielle, d'un pouls, d'une concentration en oxygène dans le sang, et d'un électrocardiogramme en tant qu'informations biologiques, dans lequel la section d'enregistrement est en outre configurée afin d'enregistrer les informations biologiques sélectionnées par la section de sélection dans la section de stockage (3e) de telle sorte que les informations biologiques soient associées au signal d'image endoscopique.

5. Système selon la revendication 1, comprenant en outre une section d'instruction (24d) configurée pour indiquer que le signal d'image endoscopique doit être capturé, dans lequel la section d'enregistrement est configurée afin d'enregistrer, dans la section de stockage (3e), les informations biologiques et le signal d'image endoscopique capturés selon une instruction de la section d'instruction (24d).

6. Produit de programme informatique destiné à gérer un système d'acquisition d'images endoscopiques afin d'enregistrer des informations relatives à l'état d'un corps examiné lors d'un examen endoscopique et des informations d'images endoscopiques, comprenant des parties de codes logiciels permettant d'effectuer les étapes suivantes dans un système selon la revendication 1 :
une première étape de communication (S57) consistant à capturer, à partir d'un endoscope (2), un signal d'image endoscopique obtenu pendant l'examen d'un corps examiné à l'aide d'un endoscope (5) ;
une seconde étape de communication (S55) consistant à capturer, à partir d'un appareil de mesure (4), les informations biologiques obtenues pendant la mesure du corps examiné observé par l'endoscope (5) ; et
une étape d'enregistrement consistant à enregistrer, dans une section de stockage (3e), les informations biologiques et le signal d'image endoscopique ainsi capturés en tant que données d'examen relatives au corps examiné de telle sorte que les informations biologiques et le signal d'image endoscopique soient associés sur la base d'informations préenregistrées identifiant le corps examiné ;
**caractérisé en ce que** l'étape d'enregistrement enregistre les informations biologiques à des intervalles périodiques prédéterminés qui peuvent être définis en vue de l'examen.

7. Produit de programme informatique selon la revendication 6, comprenant en outre des parties de codes logiciels permettant d'effectuer une étape de création de rapports (S7) consistant à créer un rapport qui comprend les informations biologiques et les données d'images endoscopiques basées sur le signal d'image endoscopique, sur la base des données d'examen enregistrées dans la section de stockage (3e).

8. Produit de programme informatique selon la revendication 6 ou 7, comprenant en outre des parties de codes logiciels permettant d'effectuer :
une étape de définition de valeurs limite (S34, S35) consistant à définir des valeurs limite pour les informations biologiques capturées ;
une étape de comparaison consistant à comparer les valeurs limite et les valeurs des informations biologiques capturées ; et
une étape de notification consistant à fournir une notification selon un résultat de comparaison de l'étape de comparaison.

9. Produit de programme informatique selon la revendication 6 ou 7, comprenant en outre des parties de codes logiciels permettant d'effectuer une étape de sélection (S30) consistant à sélectionner au moins l'un d'une pression artérielle, d'un pouls, d'une concentration en oxygène dans le sang, et d'un électrocardiogramme en tant qu'informations biologiques, dans lequel l'étape d'enregistrement enregistre les informations biologiques sélectionnées par l'étape de sélection dans la section de stockage (3e) de telle sorte que les informations biologiques soient associées au signal d'image endoscopique.
